# EUROPÄISCHE PATENTANMELDUNG

(11) **EP 0 779 269 A1**
(43) Veröffentlichungstag der Anmeldung: **18.06.1997**
(21) Anmeldenummer: 96119322.4
(22) Anmeldetag: 03.12.1996
(51) Int. Cl.: C07C 69/743, C07C 67/08

(54) **Verfahren zur Herstellung synthetischer Pyrethroide durch azeotrope Veresterung**

(30) Priorität: 15.12.1995 DE 19546920
(71) Anmelder: BAYER AG, 51368 Leverkusen (DE)
(72) Erfinder: Decker, Matthias, Dr., 50674 Köln (DE); Esser, Michael, Dr., 51373 Leverkusen (DE); Littmann, Martin, Dr., 51375 Leverkusen (DE); Sehnem, Hans-Peter, 58332 Schwelm (DE)

(57) **Zusammenfassung**

Die Erfindung betrifft ein neues Verfahren zur Herstellung synthetischer Pyrethroide der allgemeinen Formel (I) 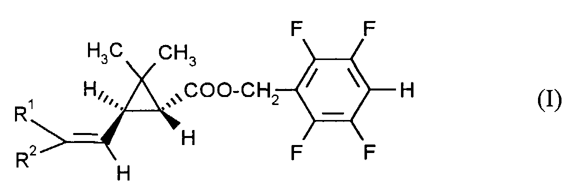 in welcher
R¹ und R² die in der Beschreibung genannten Bedeutungen haben,
basierend auf einer azeotropen Veresterung von 2,3,5,6-Tetrafluorbenzylalkohol mit der entsprechenden Carbonsäure.

## Beschreibung

Die vorliegende Erfindung betrifft ein neues Verfahren zur Herstellung von synthetischen Pyrethroiden der Formel (I) 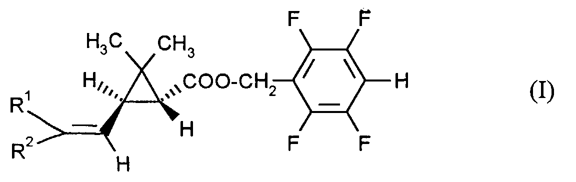 in welcher
R¹ für Methyl, Difluormethyl, Trifluormethyl oder Chlor steht und
R² für Methyl, Difluormethyl, Trifluormethyl oder Chlor steht durch azeotrope Veresterung.

Verbindungen dieses Typs haben insbesondere in der Bekämpfung von Insekten Bedeutung erlangt.

Aus DE 37 05 224 ist die Umsetzung des Säurechlorids der (+)-trans-Permethrinsäure mit 2,3,5,6-Tetrafluorbenzylalkohol nach folgendem Reaktionsschema bekannt:

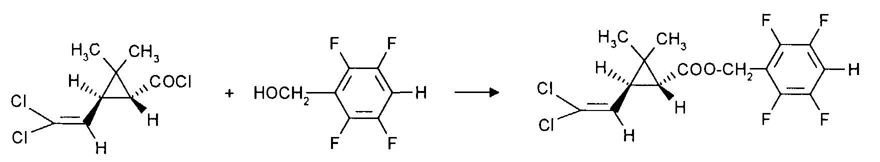

Dabei wird der (+)-trans-Permethrinsäure-D-menthylester in Methanol/Natronlauge verseift. Nach Abdestillieren des Methanols wird Wasser zugegeben und das D-Menthol mit Toluol extrahiert. Nach Ansäuern der wäßrigen Phase wird die (+)-trans-Permethrinsäure mit Toluol extrahiert.

Die toluolische Lösung wird andestilliert, bis sie wasserfrei ist und anschließend mit Thionylochlorid versetzt. Dann wird Toluol und überschüssiges Thionylchlorid abdestilliert; im Sumpf bleibt (+)-trans-Permethrinsäurechlorid zurück.

Das Säurechlorid wird dem vorgelegten Tetrafluorbenzylalkohol zugepumpt und reagiert dabei zum Transfluthrin. Das Produkt wird zunächst einer Wasserdampfdestillation unterworfen, dann mit Toluol verdünnt und die toluolische Lösung mit Kalilauge zur Hydrolyse des Permethrinsäureanhydrids ausgerührt. Nach der Phasentrennung wird Toluol abdestilliert und das Produkt kann abgefüllt werden.

Weiterhin ist aus DE 37 05 224 bekannt, daß man den Wirkstoff 2,3,5,6-Tetrafluorbenzyl-(+)-1R-trans-2,2-dimethyl-3-(2,2-dichlorvinyl)-cyclopropan-carboxylat (Transfluthrin) durch Umsetzung von Salzen der (+)-trans-Permethrinsäure mit 2,3,5,6-Tetrafluorbenzylchlorid nach folgendem Schema erhält:

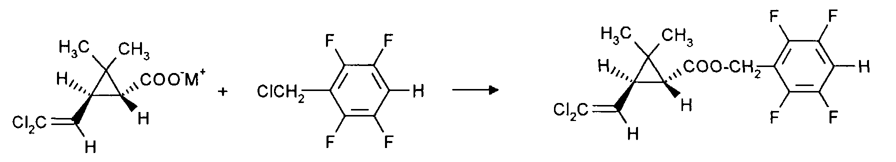

Beispielsweise verwendet man das Natriumsalz der Permethrinsäure und setzt dieses mit dem 2,3,5,6-Tetrafluorbenzylchlorid, erhältlich aus dem entsprechenden Benzylalkohol mit beispielsweise SOCl₂, um.

Schließlich ist aus der EP 378 026 allgemein ein Verfahren bekannt, wonach Verbindungen der Formel (II) 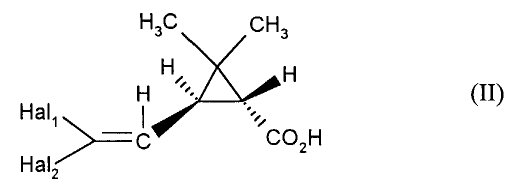 in welcher
- Hal₁ und Hal₂: gleich oder verschieden sein können und für Fluor, Chlor, Brom oder Iod stehen,
mit Benzylalkoholen der Formel 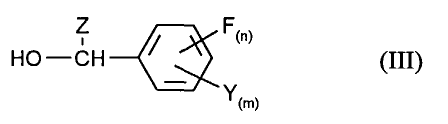 in welcher
- n: für 1 bis 5 steht und
- Y: u.a. für Wasserstoff stehen kann,
verestert werden.

Nachteilig an allen Verfahren ist die aufwendige Vorbereitung der einzusetzenden Komponenten, die zumeist in Vorstufen präpariert werden müssen und die demzufolge niedrige Gesamtausbeute an synthetischem Pyrethroid. Darüberhinaus nachteilig an dem im Stand der Technik beschriebenen Verfahren ist das Auftreten toxischer Nebenprodukte, insbesondere dem Anhydrid der jeweils eingesetzten Carbonsäure.

Es wurde nun gefunden, daß man synthetische Pyrethroide der allgemeinen Formel (I) 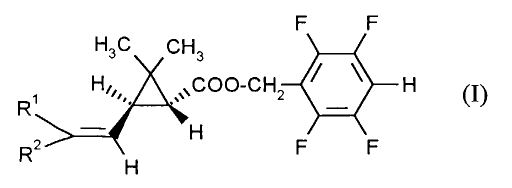 in welcher
- R¹: für Methyl, Difluormethyl, Trifluormethyl oder Chlor steht und
- R²: für Methyl, Difluormethyl, Trifluormethyl oder Chlor steht,
durch azeotrope Veresterung entsprechender Carbonsäuren der allgemeinen Formel (II) 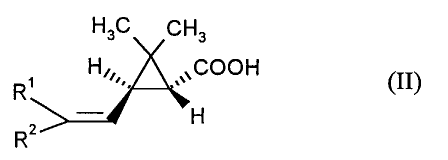 in welcher
- R¹ und R²d: die oben genannten Bedeutungen haben, mit 2,3,5,6-Tetrafluorbenzylalkohol herstellen kann.

Die Vorteile dieses Verfahrens liegen in der einfachen Durchführung begründet. Die Carbonsäuren der Formel (II) müssen nicht zum Säurechlorid, bzw. der 2,3,5,6-Tetrafluorbenzylalkohol muß nicht zum entsprechenden Chlorid umgesetzt werden. Desweiteren können während der Umsetzung keine Anhydride der entsprechenden Carbonsäure entstehen, die zumeist ungünstige Eigenschaften aufweisen und demzufolge mit hohem Aufwand entsorgt werden müssen.

Insbesondere eignet sich das erfindungsgemäße Verfahren zur Synthese des Transfluthrins (= 2,3,5,6-Tetrafluorbenzyl-(+)-1R-trans-2,2dimethyl-3-(2,2-dichlorvinyl)-cyclopropan-carboxylat).

Dabei wird die toluolische Lösung der (+)-trans-Permethrinsäure vorgelegt, mit 5 bis 7 mol-% Schwefelsäure versetzt und zum Rückflußsieden erhitzt. Unter Wasserauskreisen werden 80 mol-% Tetrafluorbenzylalkohol zudosiert und die Reaktionsmischung weitere 4 h am Rückflußsieden gehalten.

Die Reaktionslösung wird abgekühlt, mit Natronlauge der Überschuß an Säure als Natriumsalz extrahiert und nach der Verseifung wieder eingesetzt.

Schließlich wird das Toluol abdestilliert und der im Sumpf verbleibende Wirkstoff abgefüllt.

Mit dem vorliegenden erfindungsgemäßen Verfahren der azeotropen Veresterung erhöht man einerseits die Ausbeute, erzielt darüber hinaus kürzere Taktzeiten und somit insgesamt höhere Monatsausbeuten.

Eine Gegenüberstellung der Verfahren verdeutlicht dies:

Bisheriges Verfahren (A) gemäß DE 37 05 224

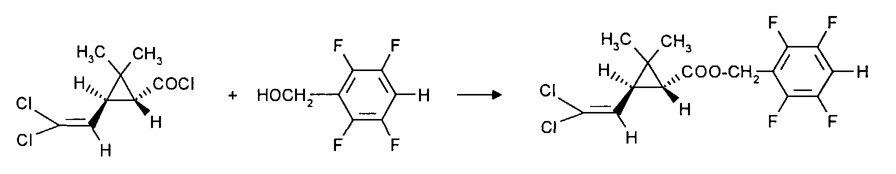

Ausbeute 83 %, Taktzeit 38 Stunden bei diskontinuierlicher Fahrweise, Kapazität 18,5 Monatstonnen.

Erfindungsgemäßes Verfahren (Azeotropdestillation); Beispiel Transfluthrin

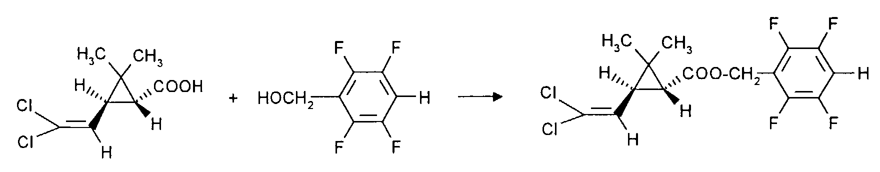

Ausbeute 93 %, Taktzeit 18 Stunden bei diskontinuierlicher Fahrweise, Kapazität 22 Monatstonnen.

Das erfindungsgemäße Verfahren wird in Gegenwart eines für azeotrope Veresterungsverfahren geeigneten Lösungsmittels durchgeführt. Als Beispiele für diese Lösungsmittel seien Toluol und Benzol genannt.

Die Reaktionstemperaturen richten sich dabei nach dem Siedepunkt des zu verwendenden Lösungsmittels.

Das erfindungsgemäße Verfahren wird vorzugsweise bei Normaldruck durchgeführt.

Das erfindungsgemäße Verfahren wird vorzugsweise mit einem Katalysator durchgeführt. Als Katalysatoren werden bevorzugt p-Toluolsulfonsäure, saurer Ionenaustauscher oder Schwefelsäure eingesetzt, insbesondere jedoch Schwefelsäure verwendet.

Beim erfindungsgemäßen Verfahren werden vorzugsweise äquimolare Mengen Carbonsäure und 2,3,5,6-Tetrafluorbenzylalkohol eingesetzt. Es konnte jedoch gezeigt werden, daß ein bis zu 1,5-facher Überschuß Carbonsäure sich durchaus positiv auf den Verfahrensablauf auswirkt (Molverhältnis: 1,5 mol Carbonsäure : 1 mol 2,3,5,6-Tetrafluorbenzylalkohol).

### Experimenteller Teil

376,2 g (1,8 mol) (+)-trans-Permethrinsäure werden in 860 g Toluol vorgelegt und mit 8 g Schwefelsäure versetzt. Der Ansatz wird zum Rückflußsieden erhitzt und während ca. 1 h 270 g (1,5 mol) 2,3,5,6-Tetrafluorbenzylalkohol in 116,0 g Toluol zudosiert; gleichzeitig wird Wasser ausgekreist. Der Ansatz wird noch 4 h am Rückflußsieden gehalten und dabei das Reaktionswasser ausgekreist.

Die Reaktionsmischung wird auf Raumtemperatur abgekühlt und zum Entfernen des Überschusses an Säure mit 300 ml 2-molarer Natronlauge extrahiert.

Die organische Phase wird mit 500 ml Wasser gewaschen und vom Lösungsmittel befreit. (Der Rückstand wird mit 250 ml Wasser vermischt und durch Abdestillieren des Wassers werden wasserdampfflüchtige Nebenkomponenten entfernt).

Ausbeute: 539,6 g ≅ 97,0 % Transfluthrin

## Patentansprüche

1. Verfahren zur Herstellung von synthetischen Pyrethroiden der allgemeinen Formel (I) 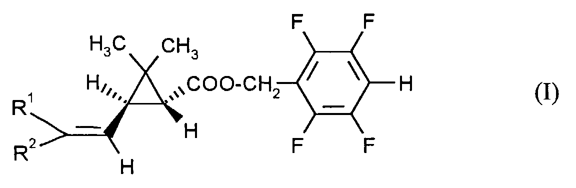 in welcher
R¹ für Methyl, Difluormethyl, Trifluormethyl oder Chlor steht und
R² für Methyl, Difluormethyl, Trifluormethyl oder Chlor steht,
dadurch gekennzeichnet, daß man Verbindungen der Formel (II) 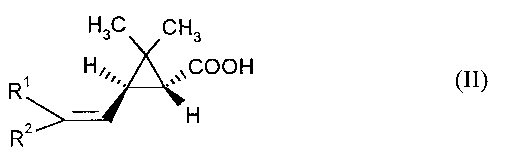 in welcher
R¹ und R² die oben genannten Bedeutungen haben,
mit 2,3,5,6-Tetrafluorbenzylalkohol azeotrop verestert.

2. Verfahren zur Herstellung von 2,3,5,6-Tetrafluorbenzyl-(+)-1R-trans-2,2-dimethyl-3-(2,2-dichlorvinyl)cyclopropan-carboxylat durch azeotrope Veresterung von (+)-trans-Permethrinsäure mit 2,3,5,6-Tetrafluorbenzylalkohol.

3. Verfahren zur Herstellung von synthetischen Pyrethroiden gemäß Anspruch 1, dadurch gekennzeichnet, daß für azeotrope Veresterungen geeignete Lösungsmittel eingesetzt werden.

4. Verfahren zur Herstellung von synthetischen Pyrethroiden gemäß Anspruch 1, dadurch gekennzeichnet, daß als Lösungsmittel Toluol eingesetzt wird.

5. Verfahren zur Herstellung von synthetischen Pyrethroiden gemäß Anspruch 1, dadurch gekennzeichnet, daß dieses unter Normaldruck durchgeführt wird.

6. Verfahren zur Herstellung von synthetischen Pyrethroiden gemäß Anspruch 1, dadurch gekennzeichnet, daß man 1 bis 1,5 mol Carbonsäure mit 1 mol 2,3,5,6-Tetrafluorbenzylalkohol azeotrop verestert.

7. Verfahren zur Herstellung von synthetischen Pyrethroiden gemäß den Ansprüchen 1 bis 6, dadurch gekennzeichnet, daß das Verfahrensprodukt frei von Anhydriden der jeweils eingesetzten Carbonsäuren ist.

8. Verfahren zur Herstellung von 2,3,5,6-Tetrafluorbenzyl-(+)-1R-trans-2,2-dimethyl-3-(2,2-dichlorvinyl)-cyclopropan-carboxylat, dadurch gekennzeichnet, daß man eine Mischung aus (+)-trans-Permethrinsäure, Toluol und Schwefelsäure zum Rückflußsieden erhitzt, mit einer Mischung von 2,3,5,6-Tetrafluorbenzylalkohol in Toluol versetzt, den gesamten Ansatz azeotrop verestert, schließlich die Reaktionsmischung auf Raumtemperatur abkühlt und überschüssige Säure sowie Lösungsmittel entfernt.
